# EUROPEAN PATENT APPLICATION

(11) **EP 4 431 099 A1**
(43) Date of publication of application: **18.09.2024**
(21) Application number: 22893086.3
(22) Date of filing: 28.10.2022
(51) Int. Cl.: A61K 31/575, A61P 31/12, A61P 31/16, A23L 33/10

(54) **COMPOSITION CONTAINING TAURODEOXYCHOLIC ACID OR PHARMACEUTICALLY ACCEPTABLE SALT THEREOF AS ACTIVE INGREDIENT FOR PREVENTING OR TREATING VIRAL INFECTIOUS DISEASES**

(30) Priority: 09.11.2021 KR 20210152864
(71) Applicant: Shaperon Inc., Seoul 06373 (KR); Seoul National University R & DB Foundation, Seoul 08826 (KR)
(72) Inventor: SEONG, Seung Yong, Seoul 03080 (KR)
(74) Representative: Vossius & Partner Patentanwälte Rechtsanwälte mbB
(86) International application number: PCT/KR2022/016721
(87) International publication number: WO 2023/085664

(57) **Abstract**

The present invention relates to a composition for preventing or treating virus infection diseases containing taurodeoxycholic acid or a pharmaceutically acceptable salt thereof as an active ingredient. Specifically, inflammatory cells and inflammatory cytokines were found to decrease in the lungs of an influenza virus-infected animal model to which taurodeoxycholic acid or a pharmaceutically acceptable salt thereof was administered as an inflammasome inhibitor, and thus the inflammasome inhibitor containing taurodeoxycholic acid or a pharmaceutically acceptable salt thereof can be used as an active ingredient in the composition for preventing or treating virus infection diseases.

## Description

### [FIELD OF THE INVENTION]

The present invention relates to a composition for the prevention or treatment of virus infection diseases containing taurodeoxycholic acid or a pharmaceutically acceptable salt thereof as an active ingredient. More specifically, the present invention pertains to a composition for the prevention or treatment of virus infection diseases containing a inflammasome inhibitor comprising taurodeoxycholic acid or a pharmaceutically acceptable salt thereof as an active ingredient.

### [BACKGROUND]

Viral diseases encompass a wide spectrum of illnesses, each manifesting distinct symptoms depending on the type of virus. Viruses causing respiratory infections include influenza viruses, coronaviruses, and rhinoviruses, while those leading to gastrointestinal conditions such as diarrhea include rotaviruses and enteroviruses. Retroviruses are associated with immune disorders, and adenoviruses, papillomaviruses, hepatitis B and C viruses are linked to cancer. Additionally, herpesviruses can cause eruptions on the face or genital skin.

Influenza virus, which causes respiratory diseases among humans, belongs to the Orthomyxoviridae family, a group of RNA viruses. It is classified into three types: A, B, and C. Among these, Type A infects mammals such as humans and pigs, as well as various species of poultry and wild birds. The natural hosts for Type A influenza viruses are known to be wild waterfowl such as ducks. Epidemiological studies on influenza infection in wild birds have confirmed the presence of 16 subtypes of hemagglutinin (HA) and 9 subtypes of neuraminidase (NA) influenza viruses among wild avian populations.

The influenza virus undergoes periodic slight variations, infecting over 10% of the population each year, predominantly during late autumn to winter. This leads to increased mortality rates, particularly among the elderly and those with chronic illnesses. Additionally, every 10 to 40 years, significant antigenic shifts occur, resulting in the emergence of new viruses that most of the population has not previously encountered. As a result, most individuals lack immunity to the new virus, facilitating person-to-person transmission and causing widespread infection, with mortality rates escalating during severe pandemics affecting over 20 to 50% of the population.

Furthermore, due to the high likelihood of significant damage if influenza viruses originating from animals mutate to become transmissible to humans, controlling influenza involves direct and indirect measures related to controlling influenza viruses infecting animals. To prevent the aforementioned damages, active global efforts are underway for the development of therapeutics and vaccines, which are being successfully commercialized and applied to both humans and animals.

The overactivation of the NLRP3 inflammasome due to P2X7 receptor activation following virus infection is a major cause of pneumonia as a respiratory disease. P2X7 receptors are distributed systemically in immune cells, pulmonary epithelial cells, and central nervous system cells. The proliferation of the virus induces cell death, leading to the release of ATP, which activates P2X7 receptors, triggering excessive activation of the NLRP3 inflammasome in the respiratory system and thereby progressing to severe illness.

The P2X7 receptor is an ion channel receptor composed of complexes belonging to the P2Xn purinergic receptor family. Stimulation of P2X7 promotes the extracellular release of potassium ions and facilitates the rapid inward movement of calcium and sodium ions into the cell. In diseased states such as infection, extracellular ATP concentrations significantly increase, leading to P2X7 receptor activation, which triggers the efflux of potassium ions and intracellular ion imbalance, promoting the NLRP3 inflammasome. Consequently, pro-IL-1β/18 is activated by the NLRP3 inflammasome, exacerbating cell death and inflammation in the surrounding tissue.

Despite detailed understanding of the mechanism of NLRP3 inflammasome activation by P2X7, P2X7 antagonists have not yet been developed. The diversity of P2X7 isoforms within individuals and the polymorphism of P2X7 between humans serve as significant barriers to the development of P2X7 antagonists. Additionally, GPCR19 forms complexes with P2X7 receptors on the cell membrane in keratinocytes, T cells, and mast cells, among others. The expression of GPCR19 decreases significantly in inflamed cells, while the expression of P2X7 receptors increases significantly, leading to amplified inflammatory responses.

Currently, amantadine, rimantadine, zanamivir, and oseltamivir are used as therapeutic agents for influenza virus infections. However, the emergence of resistant strains and the severe side effects associated with the currently available antiviral agents, such as vomiting and dizziness, require careful consideration in their application. Additionally, post-influenza virus infection, viral neutralization or inhibition of replication is necessary for viral clearance, but it is also essential to regulate the exacerbation of inflammatory responses in the respiratory tract, as described above.

The inventors have endeavored to develop a safe and low side-effect treatment for viral infectious respiratory diseases, such as influenza virus infections, which induce excessive inflammation in the respiratory tract. As a result of their efforts, they have confirmed a reduction in inflammatory cells and inflammatory cytokines in the lungs of influenza virus-infected mouse models treated with pharmacologically acceptable doses of taurodeoxycholic acid. Therefore, by demonstrating the potential use of taurodeoxycholic acid or its pharmacologically acceptable salts as inhibitors of the NLRP3 inflammasome, aimed at regulating inflammatory responses to alleviate the severity of respiratory symptoms caused by virus infections, the inventors have arrived at this patent application. This disclosure highlights the potential utility of thauridineoxycholic acid or its pharmacologically acceptable salts as effective components of compositions for the prevention or treatment of virus infection diseases.

### [Prior Art Documents]

### [Non-Patent Literature]

J Rello and A Pop-Vicas: Clinical review: Primary influenza viral pneumonia. Critical Care 2009, 13:235.

### [Detailed Description of the Invention]

### [Technical Challenges]

Virus infection diseases, such as influenza virus infections, which induce excessive inflammation in the respiratory tract, can progress severely due to excessive inflammatory responses in the respiratory system. Therefore, there is a need to regulate respiratory tract inflammation to alleviate this condition.

Accordingly, the objective of the present invention is to provide compositions for the prevention, treatment, or improvement of virus infection diseases by regulating inflammatory responses in the respiratory tract.

### [Technical Solution]

To achieve the objectives of the present invention, provided are a pharmaceutical composition for the prevention or treatment of virus infection diseases comprising an inflammasome inhibitor as an effective ingredient.

Additionally, the present invention provides a health functional food composition for the prevention or improvement of virus infection diseases comprising an inflammasome inhibitor as an effective ingredient.

Moreover, the present invention offers a method for the prevention or treatment of virus infection diseases involving administering an inflammasome inhibitor to an individual.

Furthermore, the present invention provides the use of an inflammasome inhibitor for the pharmaceutical composition for the prevention or treatment of virus infection diseases.

Additionally, the present invention offers the use of an inflammasome inhibitor for the health functional food composition for the prevention or improvement of virus infection diseases.

Additionally, the present invention provides the use of an inflammasome inhibitor for the manufacture of the pharmaceutical composition for the prevention or treatment of virus infection diseases.

Furthermore, the present invention offers the use of an inflammasome inhibitor for the manufacture of the health functional food composition for the prevention or improvement of virus infection diseases.

### [Advantages of the Invention]

The inventors administered pharmacologically acceptable salts of taurodeoxycholic acid to influenza virus-infected mouse models with the aim of alleviating severe manifestations of virus infection by regulating inflammatory responses through the inhibition of NLRP3 inflammasome activation. As a result, they observed a reduction in inflammatory cells and inflammatory cytokines in the lungs, indicating the potential of the aforementioned inflammasome inhibitors comprising taurodeoxycholic acid or its pharmacologically acceptable salt as effective components of compositions for the prevention or treatment of virus infection diseases.

### [Brief Description of the Drawings]

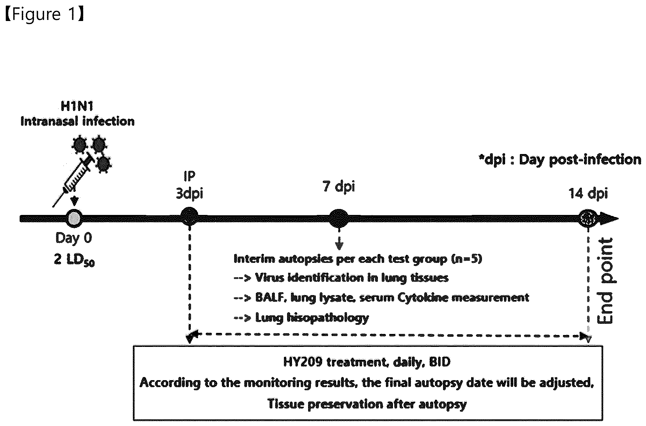

Figure 1 illustrates a schematic representation of creating an influenza virus-infected animal model and administering sodium taurodeoxycholate, referred to as sodium taurodeoxycholate (HY209), to the aforementioned animal model.

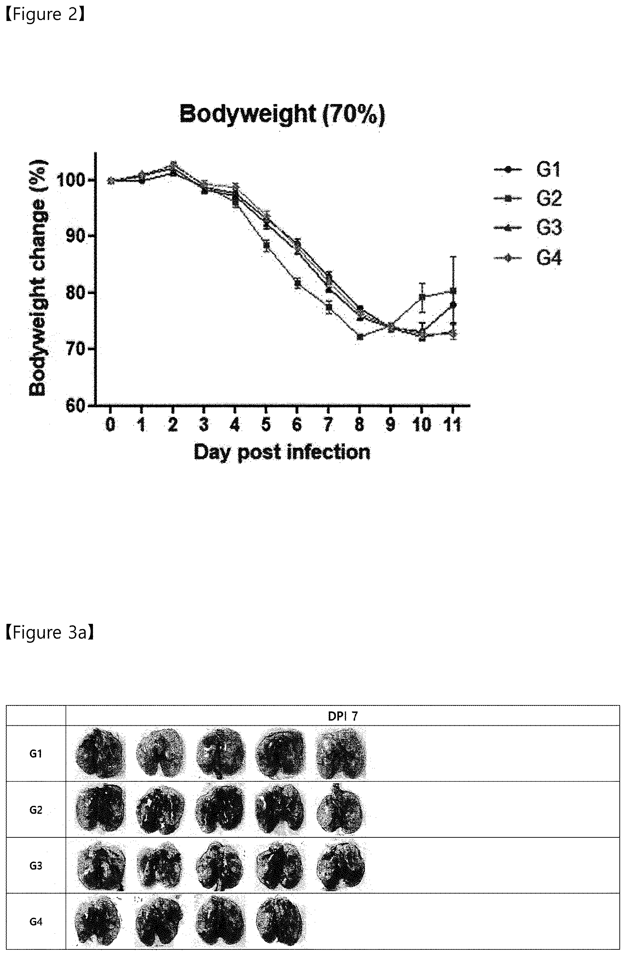

Figure 2 depicts the confirmation of body weight changes in an influenza virus-infected animal model following the administration of HY209

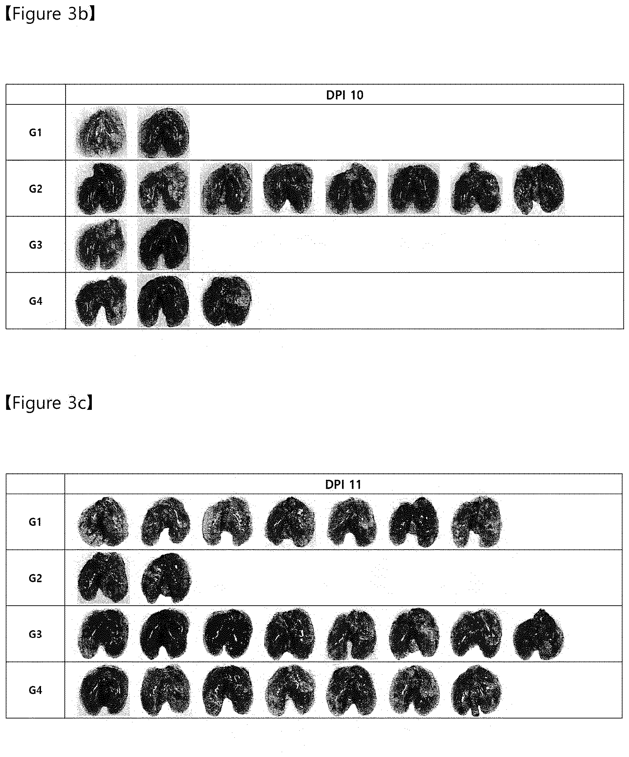

Figures 3a to 3c depict visual confirmation of lung lesions in an influenza virus-infected animal model following the administration of HY209. Specifically, Figure 3a represents lung lesions on day 7 post-infection (DPI 7), Figure 3b represents lung lesions on day 10 post-infection (DPI 10), and Figure 3c represents lung lesions on day 11 post-infection (DPI 11).

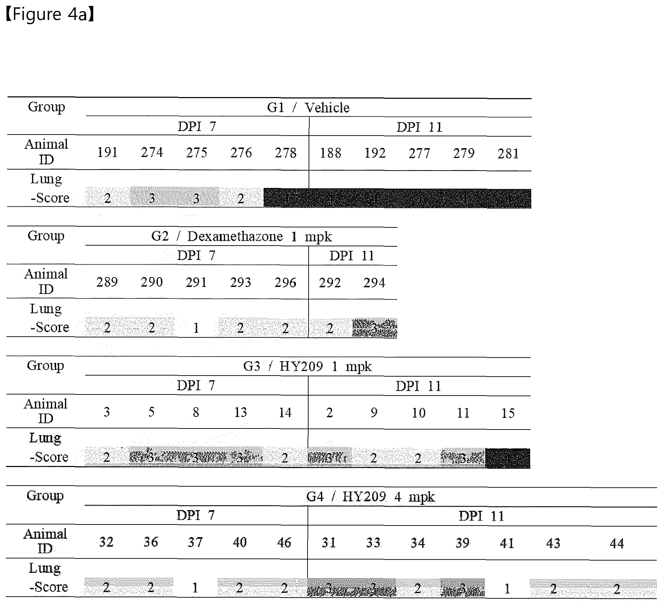

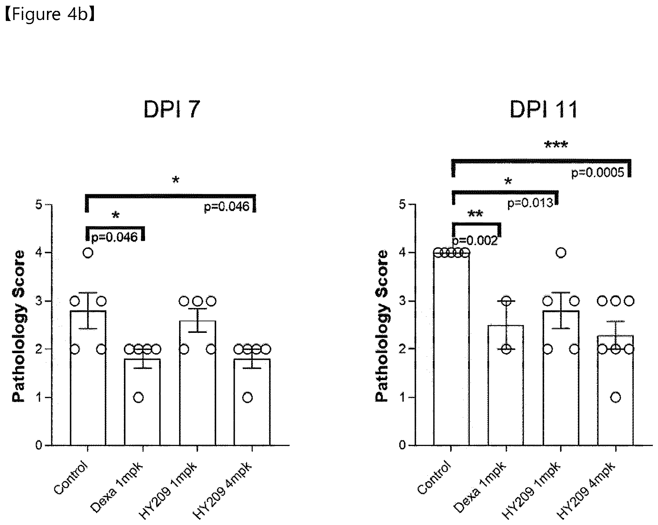

Figures 4a and 4b illustrate the results of histopathological analysis of lung tissues in an influenza virus-infected animal model following the administration of HY209. Figure 4a represents the scoring of pathological findings according to scoring criteria, while Figure 4b represents the graphical representation of the same.

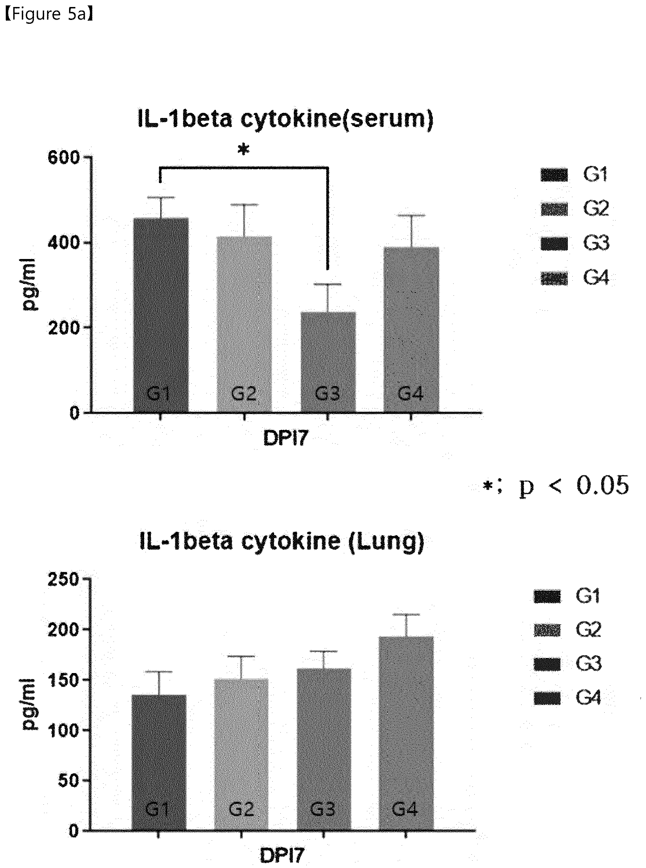

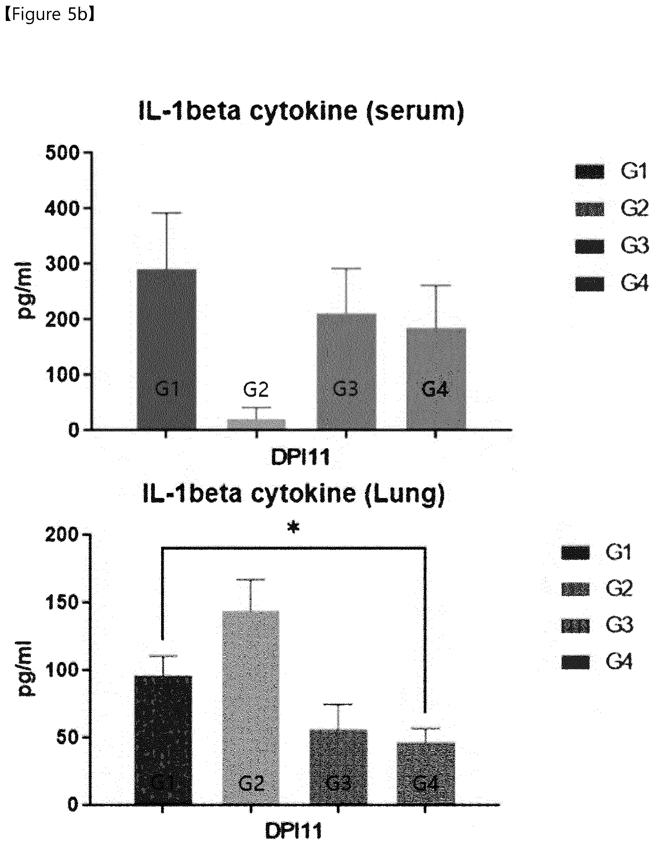

Figures 5a and 5b depict the changes in inflammatory cytokine IL-1β in serum and lungs of an influenza virus-infected animal model following the administration of HY209. Specifically, Figure 5a represents the changes in inflammatory cytokine IL-1β on day 7 post-infection (DPI 7), while Figure 5b represents the changes in inflammatory cytokine IL-1β on day 11 post-infection (DPI 11) in serum and lungs.

### [Best Mode for Carrying Out the Invention]

Herein, the present invention is further elaborated.

In this invention, the term "prevention" refers to all acts of inhibiting or delaying the occurrence, spread, and recurrence of virus infection diseases through the administration of compositions of the present invention. The term "treatment" refers to all acts of symptom improvement or beneficial alteration of the aforementioned diseases through the administration of compositions of the present invention.

In the present invention, the term "administration" refers to the provision of a specified substance to a patient through any suitable method, and the route of administration of the compositions of the present invention can be orally or non-orally through any common route that can reach the target tissue. Additionally, the compositions can be administered through any device that allows the active ingredients to reach the target cells.

The present invention provides a pharmaceutical composition for the prevention or treatment of virus infection diseases comprising an inflammasome inhibitor as an effective ingredient.

Additionally, the present invention provides a method for the prevention or treatment of virus infection diseases, comprising the step of administering an inflammasome inhibitor to an individual.

Moreover, the present invention provides the use of an inflammasome inhibitor for the pharmaceutical composition for the prevention or treatment of virus infection diseases.

Additionally, the present invention provides the use of an inflammasome inhibitor for the manufacture of the pharmaceutical composition for the prevention or treatment of virus infection diseases.

In the present invention, the term "inflammasome inhibitor" refers to an NLRP3 inflammasome inhibitor, which can inhibit the formation or activation of the NLRP3 inflammasome.

Specifically, the inflammasome inhibitor can comprise taurodeoxycholic acid or its pharmaceutically acceptable salt.

The taurodeoxycholic acid is a type of bile acid, which is a taurine-conjugated deoxycholic acid, having a chemical structure represented by [Chemical Formula 1], more specifically having a chemical structure represented by [Chemical Formula 2]:

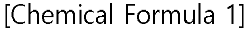

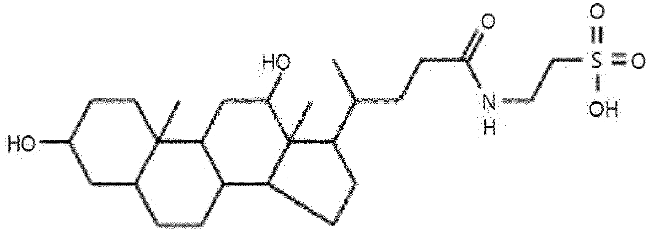

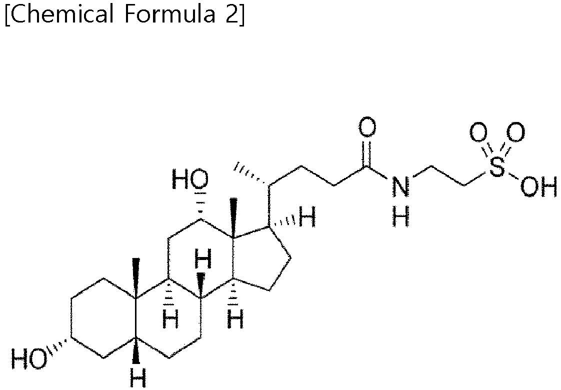

Furthermore, the taurodeoxycholic acid can be obtained from animal carcasses, such as cattle, pigs, sheep, dogs, goats, or rabbits, available commercially, or synthesized, all of which are acceptable for use.

In the present invention, the virus can be an influenza virus.

The influenza virus mentioned above is classified into influenza virus type

A, influenza virus type B, and influenza virus type C. Among these, type A is primarily known to infect humans, while type B or C is observed to infect pigs, other mammals, and various wild birds compared to type A. Additionally, it includes viruses such as avian influenza virus, swine influenza virus, and novel influenza A virus subtypes H1N1, which have recently become global concerns. Specifically, the influenza virus mentioned above can be influenza virus type A.

Moreover, the influenza virus type A can include at least one type selected from the group consisting of H1N1, H1N2, H2N2, H3N2, H5N1, H5N2, H7N2, H7N3, H7N7, H7N9, H9N2, and H10N7, specifically H1N1, without being limited thereto.

In the present invention, the virus infection diseases include, for example, influenza, common cold, novel flu, pharyngitis, bronchitis, pneumonia, enteritis, foot-and-mouth disease, viral meningitis, encephalitis, neurogenic pulmonary edema, conjunctivitis, myocarditis, hepatitis, poliomyelitis, paralysis, smallpox, variola, chronic obstructive pulmonary disease, abscess, otitis media, rinderpest, African swine fever, chickenpox, herpes, norovirus gastroenteritis, avian influenza, and swine flu, among others, induced by viruses. Specifically, it can include influenza, common cold, pharyngitis, bronchitis, or pneumonia caused by influenza virus infection, and more specifically, it can include pneumonia caused by influenza virus infection, without limitation thereto.

In the present invention, the inflammasome inhibitor can inhibit the production of inflammatory cytokines in the respiratory tract, such as IL-1β. Here, IL-1β can be generated by the activation of NLRP3 inflammasome.

The inflammasome inhibitor of the present invention encompasses pharmaceutically acceptable salts, and solvates, hydrates, racemates or stereoisomers that can be derived therefrom.

The inflammasome inhibitor of the present invention can be used in the form of pharmaceutically acceptable salts. Among these salts, the acid addition salts formed by pharmaceutically acceptable free acids are particularly useful. Acid addition salts are obtained from inorganic acids such as hydrochloric acid, nitric acid, phosphoric acid, sulfuric acid, hydrobromic acid, hydroiodic acid, nitrous acid or phosphorous acid, as well as from non-toxic organic acids such as aliphatic mono- or di-carboxylates, phenyl-substituted alkanoates, hydroxyalkanoates, alkandioates, aromatic acids, or aliphatic and aromatic sulfonic acids. Examples of such pharmaceutically non-toxic salts include sulfates, pyrophosphates, bisulfates, sulfites, bisulfites, nitrates, phosphates, monohydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butane-1,4-dioates, hexane-1,6-dioates, benzoates, chlorobenzoates, methyl benzoates, dinitrobenzoates, hydroxybenzoates, methoxybenzoates, phthalates, terephthalates, benzene-sulfonates, toluenesulfonates, chlorobenzenesulfonates, xylene-sulfonates, phenylacetates, phenylpropionates, phenyl butyrates, citrates, lactates, hydroxybutyrates, glycolates, malates, tartrates, methanesulfonates, propanesulfonates, naphthalene-1-sulfonates, naphthalene-2-sulfonates, or mandelates.

According to the present invention, acid addition salts can be prepared by conventional methods, such as dissolving the inflammasome inhibitor of the present invention in an excess of acid solution and precipitating the salt with a water-miscible organic solvent, such as methanol, ethanol, acetone, or acetonitrile. Additionally, the solvent or excess acid can be evaporated from this mixture to dryness or the precipitated salt can be isolated by suction filtration for preparation.

In addition, a pharmaceutically acceptable metal salts can be made using a base. The alkali metal or alkaline earth metal salts are obtained by dissolving the compound in an excess of alkali metal hydroxide or alkaline earth metal hydroxide solution, filtering the non-dissolved compound salt, and evaporating and drying the filtrate. In this case, it is pharmaceutically suitable to prepare a sodium, potassium, or calcium salt as the metal salt, and more specifically, in the case of taurodeoxycholic acid, it is suitable to prepare a sodium salt. In addition, a silver salt corresponding thereto is obtained by reacting the alkali metal or alkaline earth metal salt with an appropriate silver salt (e.g., silver nitrate)

When the composition is formulated, it is prepared by using diluents or excipients such as a commonly used filler, a thickener, a binder, a wetting agent, a disintegrating agent, and a surfactant.

In the present invention, the formulation can be formulated into a formulation selected from the group consisting of tablets, capsules, injections, troches, powders, granules, liquid, suspensions, oral liquids, emulsions, syrups, suppositories, vaginal tablets, and pills, but is not limited thereto and can be formulated into appropriate formulations as needed.

For example, a solid preparation for oral administration includes a tablet, a pill, a powder, a granule agent, a capsule agent, a troche agent, and the like, and such a solid preparation is prepared by mixing at least one excipient in the compound of the present invention, for example, starch, calcium carbonate, sucrose, lactose or gelatin. In addition, lubricants such as magnesium stearate or talc are also used in addition to simple excipients. Liquid preparations for oral administration include suspensions, oral liquids, emulsions or syrups, among others. Besides commonly used diluents like water and liquid paraffin, various excipients such as wetting agents, sweetening agents, flavoring agents, and preservatives can be included..

Preparations for non-oral administration include sterile solutions, non-aqueous solutions, suspensions, emulsions, lyophilized formulations, suppositories, etc.

Non-aqueous solutions, suspensions such as propylene glycol, polyethylene glycol, vegetable oils like olive oil, injectable esters like ethyl oleate can be used. As the suppository base, witepsol, macrogol, tween 61, cocoa butter, laurinji, glycerol, gelatin and the like can be employed.

The composition according to the present invention is administered in a pharmaceutically effective amount. In the present invention, the term "pharmaceutically effective amount" refers to a quantity sufficient to treat a disease with a rational ratio of benefit to risk applicable to medical therapy. The effective dosage level can be determined based on factors such as the type and severity of the patient's condition, the activity of the drug, the sensitivity to the drug, the time and route of administration, elimination rate, duration of treatment, concomitant medications, and other factors well known in the medical field. The composition of the present invention can be administered as a single therapy or in combination with other therapies, either sequentially or concurrently with conventional therapies, and can be administered singly or multiply. It is important to administer the minimum amount with minimal side effects to achieve maximum efficacy, considering all of the above factors, which can be readily determined by those skilled in the art.

Specifically, the effective dose of the compound according to the present invention may vary depending on factors such as the patient's age, gender, and weight, and it may be administered daily, every other day, or divided into 1 to 3 doses per day. However, the dosage can increase or decrease depending on the route of administration, severity, gender, weight, age, etc., so the aforementioned doses do not limit the scope of the present invention in any way.

In a specific embodiment of the present invention, the inventors administered a sodium salt of taurodeoxycholic acid at low (1 mpk) or high (4 mpk) doses to a mouse model of influenza virus infection with the aim of alleviating the severity of respiratory symptoms caused by viral infection by modulating the inflammatory response through the inhibition of NLRP3 inflammasome activation. As a result, it was observed that the production of inflammatory cells and the inflammatory cytokine IL-1β in the lungs of the low or high dose treatment groups was reduced compared to the virus infection group.

Therefore, the inventors have confirmed that pharmaceutically acceptable salts of taurodeoxycholic acid, as inflammasome inhibitors, alleviate the respiratory inflammatory response induced by influenza virus infection. Thus, the inflammasome inhibitor can be utilized as an effective component of pharmaceutical compositions for the prevention or treatment of viral infections such as influenza virus.

Furthermore, the present invention provides a health functional food composition for the prevention or improvement of virus infection diseases comprising an inflammasome inhibitor as an effective ingredient.

Additionally, the present invention provides the use of an inflammasome inhibitor for the health functional food composition for the prevention or improvement of virus infection diseases.

Furthermore, the present invention provides the use of an inflammasome inhibitor for the manufacture of the health functional food composition for the prevention or improvement of virus infection diseases.

In the present invention, as the content regarding the inflammasome inhibitor and viral infection diseases remains as previously described, specific explanations will be based on the aforementioned content, and only the unique composition of the health functional food composition will be described below.

On the other hand, the inventors have confirmed that the pharmaceutically acceptable salt of taurodeoxycholic acid, as an inflammasome inhibitor, alleviates the respiratory inflammatory response induced by influenza virus infection. Therefore, the aforementioned inflammasome inhibitor can be used as an effective component of health functional food compositions for the prevention or improvement of viral infectious diseases such as influenza virus infection.

The health functional food composition of the present invention can be prepared in any one formulation selected from powder, granules, pills, tablets, capsules, candies, syrups, and beverages, but is not limited thereto.

The health functional food composition is not particularly limited as long as it can be consumed to prevent or improve a viral infectious disease. When the health functional food composition of the present invention is used as a food additive, the health functional food composition can be added as it is or used together with other food or food ingredients, and can be appropriately used according to a conventional method. The active ingredient can be appropriately used according to the purpose of use (prevention or improvement). In general, when food or beverage is prepared, it is added in an amount of 15 parts by weight or less, preferably 10 parts by weight or less, based on the health functional food composition of the present invention. However, in the case of long-term intake for the purpose of health control, the amount may be less than or equal to the above range, and since there is no problem in terms of safety, the active ingredient may be used in an amount greater than or equal to the above range. There is no particular limitation on the kind of food. Examples of foods to which the above health functional food composition can be added include meat, sausages, bread, chocolate, candies, snacks, confectionery, pizza, ramen, other noodles, gum, dairy products comprising ice cream, various soups, beverages, tea drinks, alcohol drinks, and vitamin composites, and include all health foods in a conventional sense. In addition, the health functional food composition of the present invention can be prepared as a food, especially a functional food.

The functional food of the present invention includes ingredients conventionally added in the production of the food and includes, for example, proteins, carbohydrates, fats, nutrients, and seasoning agents. For example, in the case of manufacturing a drink, natural carbohydrates or flavoring agents can be included as additional ingredients in addition to the active ingredients. The natural carbohydrates can be monosaccharides (e.g., glucose, fructose, etc.), disaccharides (e.g., maltose, sucrose, etc.), oligosaccharides, polysaccharides (e.g., dextrin, cyclodextrin, etc.) or sugar alcohols (e.g., xylitol, sorbitol, erythritol, etc.). The flavoring agent can use a natural flavoring agent (e.g., taumartin, stevia extract, etc.) and a synthetic flavoring agent (e.g., saccharin, aspartame, etc.). In addition to the health functional food composition, various nutritional agents, vitamins, electrolytes, flavoring agents, colorants, pectic acids and salts thereof, alginic acids and salts thereof, organic acids, protective colloidal thickeners, pH regulators, stabilizers, preservatives, glycerin, alcohols, carbonated agents used in carbonated beverages, and the like can be further contained.

Below, the present invention is described in detail with reference to examples.

However, the following examples are provided for illustrative purposes only, and the scope of the present invention is not limited to the examples provided.

### <Example 1> Preparation of an Influenza Virus Infection Animal Model and Administration of Sodium Taurodeoxycholate

To investigate the efficacy of taurodeoxycholic acid (TDCA), herein referred to as TDCA, as an inflammasome inhibitor in viral infection diseases, an influenza virus infection animal model was prepared. Sodium taurodeoxycholate (HY209), the sodium salt of TDCA, was administered to the animal model.

Specifically, female BLAB/C mice aged 7 weeks were purchased and allowed to acclimate for one week before the experiments commenced. The experimental animals were housed under conditions of 22±2°C temperature, 55±5% relative humidity, and a 12-hour light-dark cycle. Water was provided ad libitum, and sterilized feed was freely accessible.

Next, as shown in the schematic diagram of Figure 1, influenza virus CA04 (human H1N1) was intranasally inoculated at a concentration of 2LD₅₀ (30 *µ*ℓ /mouse), and the drug was administered by dividing into four groups as shown in [Table 1] below. The positive control group was administered dexamethasone, an anti-inflammatory drug, as a drug, and the experimental group was administered HY209 as a low dose (1 mpk) or a high dose (4 mpk) as a drug. The drug was administered intraperitoneally at a dose of 100 *µ*ℓ/mouse on the third day after virus infection. In addition, lung tissues were separated and stored after autopsy of each group on the 7th day (7 dpi), 10th day (10 dpi), and 11th day (11 dpi) of virus infection.

**[Table 1]**

| Test Group | Mice per group | Drug and Dosage | Administration Method | Virus concentration |
|---|---|---|---|---|
| Virus-infected group(G1) | 15 | PBS | I.P | 2×LD50 |
| Positive control group(G2) | 15 | Dexamethasone | I.P (DPI 3*) | |
| Low-dose experimental group (G3) | 15 | HY209 1 mg/kg | I.P (DPI 3*) | |
| High-dose experimental group (G4) | 15 | HY209 4 mg/kg | | |

| | | | | |
|---|---|---|---|---|
| *DPI(Day post infection, Administration begins 3 days after infection) | | | | |

### <Example 2> Confirmation of body weight changes following administration of HY209 in animal models infected with influenza virus

Body weight changes of each test group of <Example 1> were confirmed.

Specifically, the body weight was measured every day during the experiment of each test group in <Example 1>, and the measured body weight was checked with the start as 100%.

As a result, as shown in Figure 2, mice in the influenza virus infection group (G1) exhibited a decrease in body weight, with no significant difference in weight loss observed among the drug treatment groups (G2, G3, and G4). It was confirmed that the influenza virus infection concentration used was suitable for inducing weight loss following influenza virus infection.

### <Example 3> Identification of lung lesions following administration of HY209 in animal models infected with influenza virus

Influenza virus infection is known to cause excessive inflammation in the respiratory tract. Lung lesions were identified by separating the lungs of each of the test groups of <Example 1>.

Specifically, each of the test groups of Example 1 was autopsied on the 7th day (7 dpi), 10th day (10 dpi), and 11th day (11 dpi) of viral infection, and then the lungs were separated. The separated lung lesions were visually confirmed (Figures 3a to 3c), and lung lesions were scored according to the pathological scoring criteria of Table 2 below, and then graphed (Figures 4a and 4b).

**[Table 2]**

| Score | The degree of inflammation |
|---|---|
| 0 | No inflammation |
| 1 | Mild inflammation |
| | Inflammation cells are located at random |
| 2 | Moderate inflammation |
| | Most of the bronchi and veins are surrounded by a thin layer of inflammatory cells (1 to 5 cell thickness) |
| 3 | Marked inflammation |
| | Most of the bronchi and veins are surrounded by a thick layer of inflammatory cells (thickness of more than 5 cells) |
| 4 | Severe inflammation |
| | Complete pulmonary inflammation is evident around all veins and bronchi. |

As shown in Figures 3a to 3c, and Figures 4a and 4b, on day 7 post-infection (7 dpi), based on pathological scoring, the virus infection group (G1 or control) scored 2.8±0.84, the positive control group (G2 or Dexa 1 mpk) scored 1.8±0.45, the low-dose experimental group (G3 or HY209 1 mpk) scored 2.6±0.55, and the high-dose experimental group (G4 or HY209 4 mpk) scored 1.8±0.45. Particularly, statistically significant reduction in pathological scores compared to the virus infection group (G1) was observed in the positive control group (G2) administered with dexamethasone and the high-dose experimental group (G4) administered with HY209 4 mpk (P=0.046). Furthermore, on day 11 post-infection (11 dpi), based on pathological scoring, the virus infection group (G1) scored 4±0, the positive control group (G2) scored 2.5±0.71, the low-dose experimental group (G3) scored 2.8±0.84, and the high-dose experimental group (G4) scored 2.29±0.76. Particularly, statistically significant reduction in pathological scores compared to the virus infection group (G1) was observed in the positive control group (G2), low-dose experimental group (G3), and high-dose experimental group (G4) (P=0.002, 0.013, 0.0005).

Additionally, the virus infection group (G1) exhibited inflammatory cell infiltration and edema, and as time progressed to day 11 post-infection, the density of inflammatory cell infiltration increased, along with thickening of the alveolar walls. However, in the positive control group (G2), low-dose experimental group (G3), and high-dose experimental group (G4), an overall decrease in the number of inflammatory cells was observed.

Through the aforementioned results, it can be concluded that HY209 alleviates the pulmonary inflammation induced by influenza virus infection."

### <Example 4> Confirmation of Changes in Pulmonary Inflammatory Cytokines Following HY209 Administration in an Influenza Virus Infection Animal Model

After separating the lungs and serum of each test group mentioned in <Example 1>, changes in the inflammatory cytokine IL-1β were confirmed.

Specifically, each of the test groups of <Example 1> was autopsy performed on the 7th day (7 dpi) and 11th days (11 dpi) of viral infection, and then lungs were separated. A lung sample was obtained by homogenizing the separated lungs with 1 ml of PBS. In addition, blood collected before autopsy was centrifuged (MF-80, Hanil Science Industrial, Incheon, Korea) at 3,000 rpm for 20 minutes for serum separation. The expression levels of IL-1β in the lung sample and the serum sample were measured according to the manufacturer's procedure using a DuoSet ELISA kit (R&D system).

The results showed that, as depicted in Figure 5a and Figure 5b, at 7 days post-infection (dpi), the levels of IL-1β in serum samples decreased in all drug-treated groups (G2, G3, and G4) compared to the virus infection group (G1). Particularly, a significant decrease in IL-1β levels was observed in the low-dose experimental group (G3) treated with HY209 1 mpk

Additionally, at 11 days post-infection (dpi), the levels of IL-1β in serum samples decreased in all drug-treated groups (G2, G3, and G4) compared to the virus infection group (G1). Particularly, in lung tissue samples, a significant decrease in IL-1β levels was observed in the experimental groups (G3 and G4) treated with HY209 compared to the virus infection group (G1).

The results above indicate that HY209 suppresses the expression of inflammatory cytokines in the lungs, which are increased by influenza virus infection.

Through the results of <Example> 1 to 4, it has been confirmed that HY209 alleviates the severity of inflammation responses, particularly in the lungs, induced by influenza virus infection. Therefore, TDCA or its pharmaceutically acceptable salts can be utilized as active ingredients in compositions for the prevention or treatment of influenza virus infection.

### [Industrial Applicability]

In the present invention, it has been confirmed that inflammatory cells and inflammatory cytokines decrease in the lungs of influenza virus-infected animal models administered with an effective dose of taurodeoxyocholic acid or a pharmaceutically acceptable salt thereof, which serves as an inflammasome inhibitor. Therefore, compounds comprising taurodeoxycholic acid or its pharmaceutically acceptable salt as an inflammasome inhibitor can be utilized as active ingredients in compositions for the prevention or treatment of virus infection diseases.

## Claims

1. A pharmaceutical composition for the prevention or treatment of virus infection diseases, comprising an inflammasome inhibitor as an active ingredient.

2. The pharmaceutical composition of claim 1 for the prevention or treatment of virus infection diseases, wherein the inflammasome inhibitor comprises taurodeoxycholic acid or a pharmaceutically acceptable salt thereof.

3. The pharmaceutical composition of claim 2 for the prevention or treatment of virus infection diseases, wherein the taurodeoxycholic acid is a compound represented by the following [Chemical Formula 2].

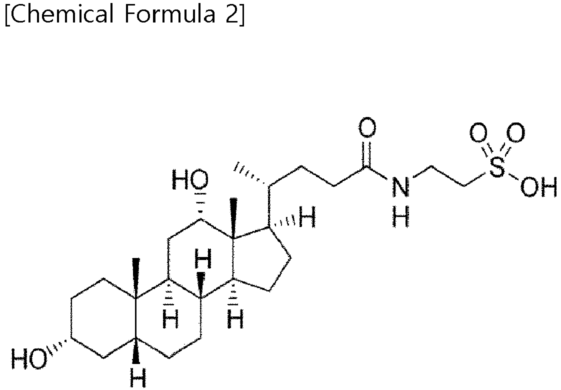

4. The pharmaceutical composition of claim 2 for the prevention or treatment of virus infection diseases, wherein the pharmaceutically acceptable salt is a sodium salt.

5. The pharmaceutical composition of claim 1 for the prevention or treatment of virus infection diseases, wherein the virus is an influenza virus.

6. The pharmaceutical composition of claim 5 for the prevention or treatment of virus infection diseases, wherein the influenza virus is selected from at least one of the group consisting of influenza virus type A, influenza virus type B, and influenza virus type C.

7. The pharmaceutical composition of claim 6 for the prevention or treatment of virus infection diseases, wherein the influenza virus type A is selected from at least one of the group consisting of H1N1, H1N2, H2N2, H3N2, H5N1, H5N2, H7N2, H7N3, H7N7, H7N9, H9N2 and H10N7.

8. The pharmaceutical composition of claim 1 for the prevention or treatment of virus infection diseases, wherein the virus infection diseases refer to influenza virus infections causing influenza, colds, pharyngitis, bronchitis, or pneumonia.

9. The pharmaceutical composition of claim 1 for the prevention or treatment of virus infection diseases, wherein the inflammasome inhibitor inhibits the generation of inflammatory cytokines in the respiratory tract.

10. The pharmaceutical composition of claim 9 for the prevention or treatment of virus infection diseases, wherein the inflammatory cytokine is IL-1β.

11. A health functional food composition for the prevention or improvement of virus infection diseases, comprising an inflammasome inhibitor as an effective ingredient.

12. A method for the prevention or treatment of virus infection diseases, comprising the step of administering an inflammasome inhibitor to an individual.

13. The use of an inflammasome inhibitor for a pharmaceutical composition for the prevention or treatment of virus infection diseases.

14. The use of an inflammasome inhibitor for a health functional food composition for the prevention or improvement of virus infection diseases.
